# EUROPÄISCHE PATENTANMELDUNG

(11) **EP 0 922 463 A2**
(43) Veröffentlichungstag der Anmeldung: **16.06.1999**
(21) Anmeldenummer: 98122621.0
(22) Anmeldetag: 27.11.1998
(51) Int. Cl.: A61K 47/34, A61K 9/16, A61K 9/20, A61K 9/24

(54) **Verfahren zur Herstellung von festen Dosierungsformen**

(30) Priorität: 01.12.1997 DE 19753299
(71) Anmelder: BASF AKTIENGESELLSCHAFT, 67056 Ludwigshafen (DE)
(72) Erfinder: Kothrade, Stephan, 67117 Limburgerhof (DE); Ernst, Andreas, 67551 Worms (DE); Sanner, Axel, 67227 Frankenthal (DE)
(74) Vertreter: Kinzebach, Werner, Dr.

(57) **Zusammenfassung**

Gegenstand der vorliegenden Erfindung ist ein Verfahren zur Herstellung von festen Dosierungsformen durch Vermischen und Aufschmelzen von mindestens einem polymeren Bindemittel, gegebenenfalls mindestens einem Wirkstoff und gegebenenfalls üblichen Additiven, Extrudieren des Gemisches und Formgebung, wobei man als polymeres Bindemittel Homo- und/oder Copolymere von Oxazolinen verwendet, die in 2-Stellung substituiert sind.

## Beschreibung

Die Erfindung betrifft ein Verfahren zur Herstellung von festen Dosierungsformen durch Vermischen von mindestens einem polymeren Bindemittel und gegebenenfalls mindestens einem Wirkstoff und gegebenenfalls üblichen Additiven unter Bildung eines plastischen u Gemisches und Formgebung. Insbesondere betrifft die Erfindung ein Verfahren zur Herstellung von festen pharmazeutischen Formen.

Die klassischen Verfahren zur Herstellung fester pharmazeutischer Formen, insbesondere Tabletten, werden diskontinuierlich durchgeführt und umfassen mehrere Stufen. Pharmazeutische Granulate stellen hierbei ein wichtiges Zwischenprodukt dar. So ist z. B. dem Buch "Pharmazeutische Technologie", Verfasser Prof. Bauer, Frömmig und Führer, Thieme Verlag, Seiten 292 ff., zu entnehmen, dass man Arzneiformen über Trockengranulierung aus der Schmelze gewinnen kann. Es wird beschrieben, dass Schmelzerstarrungsgranulate entweder durch Schmelzen und Schockerstarren, durch Ausgießen und Zerkleinern oder durch Sprüherstarren in Sprühtürmen hergestellt werden können. Ein Problem bei diesen Verfahren ist die für die Herstellung von Arzneimitteln erforderliche exakte Formgebung. Es werden häufig unregelmäßige Partikel oder Bruchstücke erzeugt, so dass die erzielte Form in keiner Weise den üblichen Arzneiformen entspricht und Granulate deshalb als eigenständige Arzneiform nur eine geringe Bedeutung besitzen. Die Herstellung der gewünschten festen Arzneiformen erfordert den Einsatz weiterer Verfahrensschritte, wie zum Beispiel die Komprimierung mit Hilfe von Tablettiermaschinen. Dies ist zeit- und kostenintensiv.

Seit einiger Zeit ist ein wesentlich einfacheres kontinuierliches Verfahren zur Herstellung fester pharmazeutischer Formen bekannt, bei dem man eine wirkstoffhaltige, lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906 und EP-A-337 256 und EP-A-358105. Damit kann eine gezielte Formgebung erreicht werden. Als polymeres Bindemittel werden insbesondere Polymere des N-Vinylpyrrolidons oder Copolymerisate davon, z. B. mit Vinylacetat, eingesetzt.

Die Verwendung von Polyoxazolinen in verschiedenen Bereichen, wie als Bindemittel in pharmazeutischen Zubereitungen ist bekannt. In der WO 95/01383, WO 95/01384, WO 95/06078 und der WO 95/06079 sind wasser- oder alkohollösliche oder dispergierbare, elastomere Copolymere offenbart, die 10 bis 60 Gew. -% Polyoxazoline mit einem Polymerisationsgrad von 10 bis 2000 als Baustein enthalten. Die Copolymere sind für Haarpflegemittel und Arzneimittel zur topischen Anwendung brauchbar.

Untersuchungen von Polyoxazolinen bezüglich Clearance und Biodistribution (Zalipsky et al., J. Pharm. Sci., 85, 133 (1996)), Toxizität (Kobayashi et al., Macromol. Chem. 184, 793 (1983)) und pharmakokinetischen Eigenschaften (Goddard et al., J. Contr. Rel. 10, 5 (1989)) wurden durchgeführt und ergaben, dass Polyoxazoline zur Herstellung von Liposomen und als Trägerpolymere für pharmazeutische Wirkstoffe brauchbar sind.

Die US-A-5 410 016 beschreibt Hydrogele aus polymerisierten und vernetzten Macromeren, die hydrophile Oligomere, z. B. Polyoxazoline, mit biologisch abbaubaren Anteilen enthalten zur Verwendung als Arzneimittelträger für Arzneiformen mit kontrollierter Freisetzung, zum zeitlich begrenzten Schutz von Geweben, zur Verhinderung von Verklebungen nach chirurgischen Eingriffen und ähnlichem.

Die WO 94/03544 beschreibt proteinverträgliche Polymerblends aus wasserlöslichen Polymeren und Matrixpolymeren und ihre Verwendung als stabile, hydrophile Oberflächen in Gefäßen zur Aufbewahrung von Proteinlösungen. Als wasserlösliches Polymer kommt auch Poly(ethyloxazolin) in Betracht.

In der WO 93/16687 sind wasserlösliche Macromere (Prepolymere) offenbart, welche mit wenigstens zwei radikalisch polymerisierbaren Substituenten modifiziert wurden. Als Macromere kommen unter anderem Poly(ethyloxazoline) in Betracht. Die Macromere werden einer Photopolymerisation unterzogen, um unter Gelbildung biologische Materialien einzukapseln.

Die US-A-5 536 505 offenbart ein Matrixsystem, das eine homogene Mischung aus Poly(2-ethyl-2-oxazolin), Celluloseacetat mit einem Substitutionsgrad von etwa 0,5 bis 3,0 und einen wasserlöslichen Wirkstoff umfasst. Das Matrixsystem erlaubt die kontrollierte Freisetzung von Wirkstoffen.

Die Verwendung von Poly(ethyloxazolin) und Poly(methacrylsäure) oder Poly(acrylsäure) zur Bildung von Polymerkomplexen, deren Wasserlöslichkeit durch elektrischen Strom gesteuert werden kann, als System zur gepulsten Wirkstofffreigabe wird von Kwon et al., Proc. 19th Int. Symp. Contr. Rel. Bioact. Mater. S. 243 (1992) und Kwon et al., Nature, 354, 291 (1991) sowie Bae et al., in "Polymeric Drugs and Drug Administration", ACS Symp. Ser. 545, 98 (1994) beschrieben.

Die WO 94/20073 offenbart Lipidpolymerkonjugate, in denen ein Vesikel bildendes Lipid kovalent mit einem wasser- und lösungsmittellöslichen Polymer verbunden ist, zur Verlängerung der Halbwertszeit von Liposomen im Blutkreislauf. Als wasser- und lösungsmittellösliches Polymer sind unter anderem Polyoxazoline brauchbar.

In der WO 92/06678 werden biologisch verträgliche Mikrokapseln beschrieben, die zur Transplantation von körperfremdem Material brauchbar sind, wobei die Mikrokapseln zur Verhinderung der Adhäsion von Zellen an der Oberfläche der Mikrokapseln eine äußere Schicht aus einem wasserlöslichen, nicht-ionischen Polymer wie Polyethylenoxid aufweisen. Anstelle von Polyethylenoxid kommen auch andere wasserlösliche Polymere wie Poly(ethyloxazolin) in Frage.

Shenouda et al., Int. J. Pharmac., 61, 127 (1990) beschreiben die teilweise Beschichtung von Hydroxypropylmethylcellulose-Kapseln mit Polyoxazolinen zur Freisetzung von Wirkstoffen aus den Kapseln mit konstanter Geschwindigkeit. Die Verwendung einer Matrix aus Hydroxypropylmethylcellulose und Poly(ethyloxazolin) führte nicht zu dieser Freisetzungscharakteristik.

Chun et al., Proc. 23rd Int. Symp. Contr. Rel. Bioact. Mater., S. 343 (1996) beschreiben die Beeinflussung von Form und Größe von Alginat-Mikrosphären durch Einbringen von oder Beschichten mit verschiedenen Substanzen, u. a. Poly(ethyloxazolin), sowie deren Einfluss auf die Freisetzung von Wirkstoffen. Mit Polymer versetzte Alginat-Mikrosphären waren kleiner als Mikrosphären aus reinem Alginat.

Die Herstellung dieser Dosierungsformen, wie Mikrokapseln und Mikropartikel sowie das Einbringen des Wirkstoffs sind jedoch sehr aufwendig und damit zeit- und kostenintensiv.

Aufgabe der Erfindung war es daher, ein einfaches und kostengünstiges Verfahren zur Herstellung von festen Dosierungsformen, insbesondere Arzneiformen, zur Verfügung zu stellen.

Überraschenderweise wurde nun gefunden, dass diese Aufgabe gelöst wird, wenn man die Dosierungsformen durch Überführen des Bindemittels in den plastischen Zustand, insbesondere durch Schmelzextrusion, herstellt und dabei Polyoxazoline als Bindemittel verwendet.

Gegenstand der vorliegenden Erfindung ist daher ein Verfahren zur Herstellung von festen Dosierungsformen durch Vermischen von mindestens einem polymeren Bindemittel, gegebenenfalls mindestens einem Wirkstoff und gegebenenfalls üblichen Additiven unter Bildung eines plastischen Gemisches und Formgebung, dadurch gekennzeichnet, dass man als polymeres Bindemittel Homo- und/oder Copolymere von 2-substituierten Oxazolinen verwendet.

Im Rahmen der vorliegenden Erfindung besitzen die nachfolgend aufgeführten Ausdrücke die folgenden Bedeutungen:

Alkyl steht für geradkettige oder verzweigte Alkylgruppen, die im Allgemeinen 1 bis 30 C-Atome aufweisen. Bevorzugt handelt es sich um C₁-C₂₁- und insbesondere C₁-C₆-Alkylgruppen. Beispiele für Alkylgruppen sind Methyl, Ethyl, n-Propyl, Isopropyl, n-Butyl, n-Hexyl, 2-Ethylhexyl, n-Nonyl, n-Dodecyl, Cetyl- und Stearyl.

Alkenyl steht für geradkettige oder verzweigte Alkenylgruppen mit 3 bis 20, insbesondere 3 bis 8 Kohlenstoffatomen. Beispiele sind Hexenyl und Oleyl.

Aryl steht vorzugsweise für Phenyl oder Naphthyl.

Acyl steht vorzugsweise für -COH oder COC₁-C₁₈-Alkyl, insbesondere COC₁-C₆-Alkyl.

Heterocyclyl steht für eine aromatische oder nichtaromatische Gruppe mit 5 oder 6 Ringatomen, die 1, 2 oder 2 Heteroatome aufweist, die unabhängig voneinander ausgewählt sind unter O, S und N. Beispiele für aromatische heterocyclische Gruppen sind Pyrrolyl, Imidazolyl, Thiazolyl, Triazolyl, Furyl, Thienyl, Oxazolyl, Isoxazolyl, Pyridyl und Pyrimidinyl. Beispiele für nichtaromatische heterocyclische Gruppen sind Pyrrolidinyl, Tetrahydrofuryl, Morpholinyl, Piperidinyl und Piperazinyl.

Das erfindungsgemäße Verfahren ermöglicht die Herstellung von festen Dosierungsformen auf einfache und kostengünstige Weise. Die vorteilhaften Eigenschaften der Polyoxazoline werden durch die Überführung in den plastischen Zustand nicht beeinträchtigt.

Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Die erfindungsgemäß erhältlichen Dosierungsformen umfassen im Allgemeinen:
a) 0 bis 99 Gew.-%, insbesondere 0,1 bis 60 Gew.-% (bezogen auf das Gesamtgewicht der Dosierungsform) eines Wirkstoffes,
b) 1 bis 100 Gew.-%, insbesondere 40 bis 99,9 Gew.-% eines polymeren Bindemittels und
c) gegebenenfalls Additive.

Wenn die Dosierungsform für Nahrungsmittelzwecke oder Futtermittelzwecke eingesetzt wird, kann der Wirkstoff fehlen, d. h. die Dosierungsform kann bis zu 100 % des polymeren Bindemittels umfassen.

Als polymeres Bindemittel werden erfindungsgemäß Oxazolin-Homopolymere und/oder Copolymere aus verschiedenen Oxazolinen oder Oxazolinen und anderen Monomeren (Comonomere) verwendet. Vorzugsweise enthalten die Copolymere mindestens 10 Gew.-%, insbesondere mindestens 20 Gew.-% und besonders bevorzugt mindestens 50 Gew.-% Oxazolineinheiten. Als Comonomere eignen sich beispielsweise monoethylenisch ungesättigte Carbonsäuren, wie Acrylsäure oder Methacrylsäure.

Die Copolymerisation von Oxazolinen ist beispielsweise in der US 4,016,192 beschrieben.

Vorzugsweise enthalten die erfindungsgemäß zur Anwendung kommenden Polymere Oxazolineinheiten der Formel 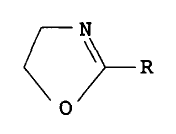 einpolymerisiert, worin R für Alkyl, das gegebenenfalls durch ein oder mehrere (mindestens 2 C-Atome voneinander entfernte) Sauerstoffatome unterbrochen ist, oder für Alkenyl, Aryl, Cycloalkyl oder Heterocyclyl steht, wobei R 1, 2 oder 3 Substituenten aufweisen kann, die unabhängig voneinander ausgewählt sind unter Alkyl, Halogen, OH, Alkoxy, Acyl, Acyloxy, COR¹, SO₂R¹, Amino, Monoalkylamino, Dialkylamino, Nitro, Aryl oder Heterocyclyl, wobei R¹ für OH, OC₁-C₆-Alkyl, NH₂, NHC₁-C₆-Alkyl oder N(C₁-C₆-Alkyl)₂ steht, oder für die oben genannten, jedoch über O, S, NR², P(OR²)₂, SiOR³, Si(R³)³ an den Oxazolinring gebundenen Substituenten steht, wobei R² für H oder C₁-C₆-Alkyl steht und die Reste R³ unabhängig voneinander für C₁-C₆-Alkyl stehen.

Bevorzugt steht R für Alkyl- und Hydroxyalkylreste mit 1 bis 30, insbesondere 1 bis 21 Kohlenstoffatomen. Insbesondere bevorzugt sind als Rest R Methyl, Ethyl, Hydroxyethyl, Hydroxypropyl, Stearyl oder Cetyl.

Ein Überblick über die Synthese von Oxazolinen und deren Polymerisation findet sich in den Henkel-Referaten 28/1992, 43-47.

Die Polyoxazoline werden in an sich bekannter Weise durch kationisch initiierte Ringöffnungspolymerisation unter Bildung von Einheiten der Formel 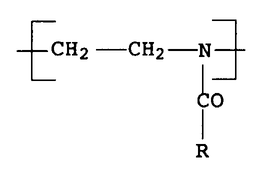 erhalten.

In der chemischen Struktur entsprechen die erfindungsgemäß als Bindemittel verwendeten Polyoxazoline N-acylierten Polyethyleniminen. Durch Hydrolyse lassen sie sich unter Abspaltung der Acylgruppe in Polyethylenimine umwandeln, die sich im Vergleich zu dem durch Polymerisation von Ethylenimin erhaltenen Polymeren durch eine hohe Linearität auszeichnen. Der Polymerisationsgrad von Polyoxazolinen kann über die eingesetzte Katalysatormenge praktisch beliebig eingestellt werden. Die Herstellung wird üblicherweise als Lösungs- oder Substanzpolymerisation mit Trifluormethansulfonsäureester oder para-Toluolsulfonsäuremethylester oder anderen bekannten, für die kationische Polymerisation geeigneten Katalysatoren durchgeführt.

Zur Herstellung der Copolymere können einerseits verschiedene Oxazoline copolymerisiert werden und andererseits Oxazoline mit anderen geeigneten Monomeren mit ausreichend hoher Nukleophilie copolymerisiert werden.

Neben den oben beschriebenen polymeren Bindemitteln können, insbesondere bis zu 30 Gew.-%, bezogen auf das Gesamtgewicht des Bindemittels, weitere Bindemittel, wie Polymere, Copolymere, Cellulosederivate und Stärke eingesetzt werden. Geeignet sind beispielsweise:

Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Das polymere Bindemittel muß in der Gesamtmischung aller Komponenten im Bereich von 50 bis 200 °C, vorzugsweise 60 bis 130 °C erweichen oder schmelzen. Die Glasübergangstemperatur der Mischung muss daher unter 200 °C, vorzugsweise unter 130 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht.

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind.

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew. -% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epiconazol und Quinmerac.

Das erfindungsgemäße Verfahren ist beispielsweise zur Verarbeitung folgender Wirkstoffe geeignet:
Acebutolol, Acetylcystein, Acetylsalicylsäure, Aciclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Insbesondere eignen sich die erfindungsgemäßen Dosierungsformen auf Grund der verhältnismäßig niedrigen Glastemperatur von 60 bis 80 °C der erfindungsgemäß als Bindemittel verwendeten Polyoxazoline sehr gut für temperaturempfindliche Wirkstoffe. Dazu zählen z. B. Enzyme, wie Pankreatin, Lipasen ...

Auch zur Herstellung von festen Dosierungsformen für temperaturempfindliche Pflanzenschutzmittel ist das erfindungsgemäße Verfahren vorteilhaft.

Zur Herstellung der festen Dosierungsformen wird ein plastisches Gemisch der Komponenten (Schmelze) bereitgestellt, das anschließend einem Formgebungsschnitt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise MischKnetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d. h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heißoder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die folgenden Beispiele sollen das erfindungsgemäße Verfahren veranschaulichen, ohne es jedoch zu beschränken.

### Beispiele

Zur Herstellung der festen Dosierungsformen wurden die angegebenen Mengen an Pflanzenschutzwirkstoff oder Arzneimittel, polymerem Bindemittel (Polyethyloxazolin; M_{w} ∼ 400 000), nicht-ionischem Tensid und ionischem Dispergiermittel entweder gemischt oder separat in einen gleichlaufenden, dichtkämmenden Doppelschneckenextruder ZSK 30 (Fa. Werner und Pfleiderer) eingebracht und über 12 Temperaturzonen extrudiert. Die Drehzahl der Schnekken wurde im Bereich von 100 bis 300 U/min variiert, die Temperatur lag im Bereich von 30 bis 80 °C in den Förderzonen und 80 bis 160 °C in den Schmelzzonen. Die genauen Bedingungen sind bei den einzelnen Beispielen angegeben. Der Durchsatz lag bei 2 bis 4 kg pro Stunde und die Verweilzeit bei 1 bis 2 Minuten. Der Wirkstoff, das Polymer und die Zusätze wurden über Dosierwagen am Extrudereingang zugefahren, gefördert und aufgeschmolzen. Gegebenenfalls wurde anschließend das nicht-ionische Tensid flüssig zugefahren und mit Misch- und Knetelementen eingearbeitet. Gewünschtenfalls wurde die Masse vor der Zugabe entgast. Die durch eine Düse aus dem Extruder ausgetragene Schmelze wurde nach dem Abkühlen gebrochen, gemahlen und durch Siebung klassiert. Die Siebfraktion mit 400 bis 500 µm wurde für die anwendungstechnischen Untersuchungen verwendet. Die Bedingungen sind in der folgenden Tabelle 1 zusammengefasst.

**Tabelle 1**

| Beispiel Nr. | Wirkstoff Gew.-% | Polyethyloxazolin Gew.-% | Lutensol AT 25^{a}) Gew.-% | Zusatz Gew.-% | Temperatur, Zonen 1-12 °C | Drehzahl U/min |
|---|---|---|---|---|---|---|
| 1 | 50 Kresoximmethyl | 50 | | | 30, 80, 90-90 | 200 |
| 2 | 50 Kresoximmethyl | 40 | 10 | | 30, 80, 85-85 | 150 |
| 3 | 50 Kresoximmethyl | 40 | 5 | 5, Tamol NH^{b}) | 30, 80, 85-85 | 150 |
| 4 | 50 Kresoximmethyl | 40 | 5 | 5, Wettol NT1^{c}) | 30, 80, 85-85 | 150 |
| 5 | 50 Kresoximmethyl | 40 | 5 | 5, Ufoxan 3A^{d}) | 30, 80, 85-85 | 150 |
| 6 | 50 Vinclozolin | 50 | | | 30, 80, 90-90 | 200 |
| 7 | 50 Vinclozolin | 40 | 10 | | 30, 80, 85-85 | 150 |
| 8 | 50 Vinclozolin | 40 | 5 | 5, Tamol NH | 30, 80, 85-85 | 150 |
| 9 | 50 Vinclozolin | 40 | 5 | 5, Wettol NT1 | 30, 80, 85-85 | 150 |
| 10 | 50 Vinclozolin | 40 | 5 | 5, Ufoxan 3A | 30, 80, 85-85 | 150 |

| | | | | | Zonen 1-7 | |
|---|---|---|---|---|---|---|
| 11 | 20 bakt. Lipase | 80 | | | 20, 50, 90, 90, 80, 80, 90 | |
| 12 | 10 Pancreatin | 90 | | | 20, 50, 90, 80-80, 95 | |

| | | | | | | |
|---|---|---|---|---|---|---|
| a) nicht-ionisches Tensid auf Basis ethoxilierter Fettalkohole, Alkylphenole, Fettamine sowie Alkylglucoside | | | | | | |
| b) Kondensationsprodukt aus Naphthalinsulfonsäure und Formaldehyd | | | | | | |
| c) Alkylnaphthalinsulfonsäure-Na-Salz | | | | | | |
| d) Ligninsulfonat | | | | | | |

1,0 g der Siebfraktionen mit 400-500 µm der Beispiele 1-10 wurden jeweils in 1 000 ml vollentsalztes Wasser gegeben. Die erhaltene Dispersion wurde 5 Minuten bei 3 U/min gerührt (IKA-Magnetrührer, RET-G, Janke & Kunkel GmbH, Staufen; 1 l-Glasgefäß ⌀ 10 cm, T = 20 °C). Anschließend wurde über ein 160 µm-Drahtsieb abfiltriert. Der Siebrückstand in Gew.-%, bezogen auf die Ausgangsmenge, betrug:

| Beispiel | Siebrückstand [%] |
|---|---|
| 1 | 8,2 |
| 2 | 2,1 |
| 3 | 1,5 |
| 4 | 2,8 |
| 5 | 1,3 |
| 6 | 5,3 |
| 7 | 0,8 |
| 8 | 0,3 |
| 9 | 0,5 |
| 10 | 1,1 |

Die Versuche belegen, dass die erfindungsgemäßen Granulate schnell und feinteilig in Wasser dispergieren. Sie sind daher für Spritz- und Streuapplikationen in der Landwirtschaft geeignet.

### Beispiel 13

50 Gew. -% Verapamil-Hydrochlorid und 50 Gew. -% Polyethyloxazolin (M_{w} ∼ 400 000) wurden unter den nachfolgend angegebenen Bedingungen extrudiert (Doppelschneckenextruder ZSK 30 der Fa. Werner und Pfleiderer) und zu 500 mg-Formen kalandriert.
- Schuss 1: 30 °C
- Schuss 2: 50 °C
- Schuss 3: 60 °C
- Schuss 4: 80 °C
- Schuss 5: 60 °C
- Düse: 60 °C

Die Freisetzung nach 1 Stunde betrug 100 % (Paddle-Modell nach USP (pH-change)).

### Beispiel 14

72 Gew.-% Paracetamol und 28 Gew.-% Polyethyloxazolin (M_{w} ∿ 400 000) wurden unter den nachfolgend angegebenen Bedingungen extrudiert (Doppelschneckenextruder ZSK 30 der Fa. Werner und Pfleiderer) und zu 500 mg-Formen kalandriert.
- Schuss 1: 30 °C
- Schuss 2: 60 °C
- Schuss 3: 80 °C
- Schuss 4: 80 °C
- Schuss 5: 80 °C
- Düse: 80 °C

Die Freisetzung nach 1 Stunde betrug 100 % (Paddle-Modell nach USP (pH-change)).

## Patentansprüche

1. Verfahren zur Herstellung von festen Dosierungsformen durch Vermischen von mindestens einem polymeren Bindemittel, gegebenenfalls mindestens einem Wirkstoff und gegebenenfalls üblichen Additiven unter Bildung eines plastischen Gemisches und Formgebung, dadurch gekennzeichnet, dass man als polymeres Bindemittel Homo- und/oder Copolymere von Oxazolinen, die in 2-Stellung substituiert sind, verwendet.

2. Verfahren nach Anspruch 1, dadurch gekennzeichnet, dass die Oxazolinhomo- oder Copolymere aufgebaut sind aus Monomeren der Formel 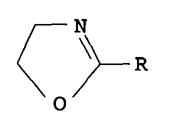 worin R für Alkyl, das gegebenenfalls durch ein oder mehrere (mindestens 2 C-Atome voneinander entfernte) Sauerstoffatome unterbrochen ist oder für Alkenyl, Aryl, Cycloalkyl oder Heterocyclyl steht, wobei R 1, 2 oder 3 Substituenten aufweisen kann, die unabhängig voneinander ausgewählt sind unter Alkyl, Halogen, OH, Alkoxy, Acyl, Acyloxy, COR¹, SO₂R¹, Amino, Monoalkylamino, Dialkylamino, Nitro, Aryl oder Heterocyclyl, wobei R¹ für OH, OC₁-C₆-Alkyl, NH₂, NHC₁-C₆-Alkyl oder N(C₁-C₆-Alkyl)₂ steht, oder R auch für die oben genannten, jedoch über O, S, NR², P(OR²)₂, SiOR³, Si(R³)³ an den Oxazolinring gebundenen Substituenten steht, wobei R² für H oder C₁-C₆-Alkyl steht und die Reste R³ unabhängig voneinander für C₁-C₆-Alkyl stehen.

3. Verfahren nach Anspruch 2, dadurch gekennzeichnet, dass R für C₁-C₃₀-Alkyl oder C₁-C₃₀-Hydroxyalkyl und insbesondere für Methyl, Ethyl, Hydroxyethyl, Hydroxypropyl, Stearyl, Cetyl oder Palmityl steht.

4. Verfahren nach einem der vorhergehenden Ansprüche, dadurch gekennzeichnet, dass die Bildung des plastischen Gemisches durch Vermischen und/oder Aufschmelzen der Komponenten in einem Extruder erfolgt.

5. Verfahren nach einem der Ansprüche 1 bis 4 zur Herstellung von pharmazeutischen wirkstoffhaltigen Dosierungsformen.

6. Verfahren nach einem der Ansprüche 1 bis 4 zur Herstellung von Pflanzenbehandlungsmitteln, Fungiziden, Herbiziden oder Insektiziden oder Mischungen davon.

7. Verfahren nach einem der Ansprüche 1 bis 4 zur Herstellung von Futtermittelzusatzstoffen und -zusätzen.

8. Verfahren nach einem der Ansprüche 1 bis 4 zur Herstellung von Nahrungsmittelzusätzen.

9. Feste Dosierungsformen, erhältlich nach einem Verfahren gemäß einem der Ansprüche 1 bis 8.

10. Verfahren zur Bekämpfung fungizider und insektizider Pathogene bei Pflanzen sowie unerwünschten Pflanzenwuchses, dadurch gekennzeichnet, dass man die Pflanzen mit einem nach Anspruch 6 erhältlichen Pflanzenbehandlungsmittel behandelt.
